# FASCICULE DE BREVET EUROPEEN

(11) **EP 0 582 530 B1**
(45) Date de publication et mention de la délivrance du brevet: **08.01.1997**
(21) Numéro de dépôt: 93420331.6
(22) Date de dépôt: 29.07.1993
(51) Int. Cl.: C04B 28/36, C04B 41/50, G01N 33/38

(54) **Composition à base de soufre, moulable à chaud, et surfaçage réalisé au moyen de cette composition**
Thermoformbare Schwefelzusammensetzung und damit hergestellte Flächenbearbeitung
Thermo-moldable sulphur compositions and surface processing based on such a composition

(30) Priorité: 03.08.1992 FR 9209805
(43) Date de publication de la demande: 09.02.1994
(73) Titulaire: LAFARGE MATERIAUX DE SPECIALITES, 92120 Montrouge (FR)
(72) Inventeur: Cavailles, Richard, F-07700 St Marcel d'Ardeche (FR); Levy, Christophe, F-92100 Boulogne (FR)
(74) Mandataire: Tilloy, Anne-Marie

(56) Documents cités:
- AT-A- 382 609
- DD-A- 203 400
- DE-A- 2 424 066
- GB-A- 2 015 568
- US-A- 4 256 499
- US-A- 4 534 225

## Description

L'invention a pour objet une composition à base de soufre moulable à chaud.

L'invention a également pour objet l'application de cette composition à la fabrication d'articles moulés, et plus particulièrement au surfaçage des éprouvettes de béton dans les essais de résistance à la compression.

Que ce soit dans le domaine de la préfabrication, du bâtiment, du génie civil ou des routes, les exigences de qualité et les niveaux de performance demandés aux bétons vont croissants. Pour la réalisation de grands ouvrages (ponts, tours, etc.) et aussi de chantiers plus classiques, on emploie de plus en plus de bétons à hautes performances (BHP), c'est-à-dire des bétons capables de développer une résistance à la compression à 28 jours au moins égale à 50 MPa. Egalement, des bétons à très hautes performances (BTHP), c'est-à-dire capables de développer une résistance à la compression à 28 jours au moins égale à 100 MPa, ont été développés dans des domaines industriels où leur résistance est un élément déterminant.

Les répercussions des propriétés des BHP et BTHP sont multiples tant sur la structure des constructions que sur la durabilité des ouvrages ou les délais d'exécution quand les bétons employés ont un niveau de performance suffisant avec une bonne régularité. Il est impératif de respecter ces exigences car sinon la pérennité de l'ouvrage obtenu risque d'être sérieusement compromise, ainsi que la sécurité des personnes devant vivre dans l'environnement de cet ouvrage.

Pour éviter d'en arriver à une telle extrémité, il est de pratique courante de contrôler régulièrement les performances des bétons utilisés sur les chantiers, notamment dans des tests de résistance à la compression, ceci afin qu'elle soit en rapport avec la nature du chantier et atteigne assez précisément la valeur exigée.

La valeur de la résistance en compression d'un béton est habituellement mesurée sur une éprouvette en béton, généralement cylindrique, que l'on soumet à une contrainte de rupture en compression.

La fiabilité de cet essai est d'autant plus grande que l'éprouvette en béton respecte certaines règles :
- homogénéité de l'éprouvette,
- un champ de contrainte au mieux uniforme ou au moins symétrique par rapport à l'axe de l'éprouvette.

Ce dernier point est atteint si les faces d'appui de l'éprouvette sont planes et perpendiculaires à l'axe et si les plateaux de la presse sont parfaitement plans.

Si ces règles ne sont pas respectées, les valeurs de résistance mesurées ne sont pas conformes à la réalité et un contrôle efficace des bétons mis en oeuvre sur un chantier n'est bien entendu plus possible.

Dans cet objectif, il importe que la planéité de ces bases mises en contact avec la presse soit la plus parfaite possible.

Différentes méthodes de préparation des éprouvettes utilisées dans les essais de résistance en compression sont actuellement bien connues.

Une première méthode consiste à rectifier, au moyen d'une meule lapidaire, les bases des éprouvettes. Bien que la planéité des surfaces rectifiées soit très bonne et permette d'atteindre des valeurs de résistance conformes à la réalité, cette méthode présente plusieurs inconvénients. Elle est tout d'abord très longue. L'appareil utile à sa mise en oeuvre représente un investissement important et, par ailleurs, nécessite une maintenance et des réglages précis. De ce fait, elle est onéreuse.

En outre, cette méthode ne peut s'appliquer qu'à des bétons ayant une résistance en compression de l'ordre de 20 MPa ou supérieure car sinon, il risque de se produire un arrachement de la matrice cimentaire.

Une deuxième méthode consiste à surfacer les bases de l'éprouvette au moyen d'un mortier de soufre qui, conventionnellement, comprend 60 % en poids de soufre et 40 % en poids de sable. Cette méthode est plus économique et plus rapide que la précédente. En outre, jusqu'à des niveaux de résistance en compression de 50 MPa, on ne constate pratiquement pas de différence entre les valeurs obtenues par les deux méthodes précitées.

En revanche, au-delà de 50 MPa, on constate un écart entre les valeurs obtenues par les deux méthodes précitées, qui s'accentue avec l'augmentation des résistances pour devenir inacceptable pour les BHP et les BTHP. En d'autres termes, les valeurs mesurées à l'aide de la deuxième méthode ne sont plus conformes à la réalité et, en outre, deviennent plus faibles.

Pour surmonter les problèmes posés par les deux méthodes précitées, on a été conduit à mettre au point d'autres méthodes de préparation des éprouvettes destinés à subir des essais d'écrasement.

A cet égard, on peut notamment citer la méthode dite de "la boîte à sable", selon laquelle une couche de sable confinée dans une boîte en acier est posée sur les deux bases de l'éprouvette (Bulletin de liaison des Laboratoires des Ponts et Chaussées ; 1989 ; No. 164 ; pages 87-88). Cette méthode offre de bons résultats.

Cette méthode est néanmoins relativement complexe aussi bien dans les moyens employés que dans sa mise en oeuvre. Elle nécessite en effet l'achat d'un bâti spécial avec vibrateur pour la mise en place des boîtes en acier et le temps pour préparer une éprouvette est assez long (de l'ordre de 20 minutes).

Egalement, pour suppléer aux difficultés occasionnées par les autres méthodes, on a proposé d'utiliser des patins en élastomère, par exemple en néoprène confiné par de l'acier. Comme la précédente méthode, celle-ci n'est pas totalement satisfaisante du fait de la complexité des moyens employés et de sa mise en oeuvre.

L'invention a donc pour principal objet des moyens ne présentant pas les inconvénients de l'art antérieur précité.

En d'autres termes, l'invention vise des moyens qui soient économiques et qui permettent d'atteindre rapidement et précisément la valeur, conforme à la réalité, de la résistance en compression d'un béton jusqu'à un niveau de compression élevé.

Pour ce faire, et de façon particulièrement surprenante, on a découvert des compositions à base de soufre moulables à chaud, qui, contrairement aux mortiers de soufre connus, garantissent l'obtention de bons résultats lors de l'évaluation des BHP et des BTHP.

Plus précisément, l'invention a pour objet une composition à base de soufre, moulable à chaud, caractérisée en ce qu'elle comprend :
- du soufre, à raison d'au moins 40 % du poids de la composition globale ;
- et des charges minérales, parmi lesquelles on trouve au moins une charge minérale possédant une dureté de l'ordre de grandeur de celle du corindon ou supérieure.

Dans un souci de simplification, dans la suite de la description, les charges minérales possédant une dureté de l'ordre de grandeur de celle du corindon ou supérieure seront désignées par "charges minérales (A)", tandis que celles possédant une dureté inférieure à celle du corindon seront désignées par "charges minérales (B)".

Avantageusement, la composition de l'invention comprend également un agent pour limiter la décantation des charges minérales quand la composition est à l'état fondu.

Afin que la composition selon l'invention soit suffisamment maniable, la teneur en soufre est avantageusement choisie inférieure ou égale à 60 % en poids de la composition totale.

De préférence, la composition selon l'invention comprend au moins 45 % en poids de soufre et, de préférence encore, 50 ± 2% en poids de soufre.

Quand la composition selon l'invention est destinée à surfacer des éprouvettes dans des essais de compression :
- la proportion des charges minérales (B) doit être limitée. Dès que les résistances en compression mesurées sur les bétons sont supérieures ou égales à 70 MPa, les charges minérales (B) sont exclues de la composition selon l'invention de façon à obtenir un comportement correct du produit de surfaçage lors de l'essai de compression.
- toujours dans le cas du produit de surfaçage, on limite la taille maximale des grains formant les charges minérales (A) et (B) à une valeur d'au plus 0,8 mm (inclus). Au-delà, en effet, les résultats des essais risquent d'être très dispersés. De préférence, cette taille maximale est au plus égale à 0,5 mm (inclus) et, de préférence encore, inférieure à 0,3 mm (inclus). De préférence, les résistances des essais de compression les meilleurs sont obtenus quand la taille maximale des grains formant les charges minérales (A) et (B) est au plus égale à 0,2 mm (inclus). Les résultats sont améliorés quant cette taille est de l'ordre de 0,1 mm.

Parmi les charges minérales (A) convenant à l'invention, on peut citer, par ordre de préférence, en partant de la charge procurant les meilleures performances :
- le corindon, dont le corindon brun qui est une alumine alpha impure,
- l'alumine tabulaire qui est une alumine alpha obtenue par frittage, la cuisson suivant une opération d'agglomération,
- l'alumine A ou alumine alpha obtenue par frittage,
- le cristalba qui est un corindon pur électro fondu,
- le carbure de silicium,
- ou des mélanges des charges minérales abrasives précitées.

Dès lors que l'on ne recherche pas des résistances en compression supérieures à environ 70 MPa (100 bars), la composition de mortier de soufre de l'invention peut comprendre des charges minérales autres que les charges minérales (A). En revanche, pour des valeurs de résistances en compression supérieures à environ 70 MPa (700 bars), les charges minérales (B) devront être exclues de la composition de mortier de soufre de l'invention et ceci uniquement dans le cas des produits de surfaçage.

Parmi les charges minérales (B) susceptibles de convenir à l'invention, on peut citer, de façon non exhaustive, le sable fin, des charges minérales fines comme le mica, des cendres volantes, les argiles, les pouzzolanes ou des mélanges des charges minérales précités.

Dans des domaines comme le moulage, le calage, le colmatage ou la réparation d'ouvrages en béton comprenant des fissures ou des cavités, la proportion en charges minérales (B) sera fonction des résistances mécaniques (en compression et en flexion/ traction) recherchées. En tout état de cause, leur proportion, dans la composition de mortier de soufre globale, sera adaptée à l'usage.

Dans certains cas, il peut être avantageux d'employer un agent permettant d'éviter la ségrégation des charges minérales, en particulier des charges minérales (A). On peut notamment utiliser les silico-aluminates connus comme permettant un maintien en suspension des particules, la proportion de cet agent étant au plus égale à 5 % en poids de la composition totale.

D'autres additifs peuvent être incorporés dans la composition de mortier de soufre de l'invention, comme par exemple un agent hydrofugeant ou un colorant, sous réserve que ces additifs n'affectent quasiment pas la viscosité de la composition et qu'ils ne soient pas vaporisables à la température de fusion du soufre ou à une température inférieure.

En revanche, on recommande, pour des raisons de sécurité, d'exclure les liquides hautement inflammables, comme le styrène à la température de fusion de soufre.

La présence des liquides ayant une température d'ébullition inférieure à la température de fusion du soufre, comme l'eau, doit être évitée car, en se vaporisant, ces liquides peuvent générer des microbulles qui vont conduire à un état de surface du mortier de soufre durci qui n'est pas impeccable.

La composition à base de soufre selon l'invention est avantageuse à plus d'un titre :
- elle est d'un emploi aisé notamment en raison de sa maniabilité que l'on améliore, si nécessaire, grâce à l'adjonction d'un agent permettant d'éviter les phénomènes de ségrégation et donc de conserver un maintien de l'homogénéité dans le temps. En effet, il suffit de soumettre la composition à base de soufre selon l'invention à une température suffisante (de l'ordre de 140°C) pour faire fondre le soufre, d'homogénéiser le liquide obtenu par agitation, puis de l'appliquer à l'état liquide sur la surface ou dans le volume considéré. Lors du refroidissement, les résistances du mortier de soufre se développent rapidement.
- elle constitue un produit prêt à l'emploi, en particulier pour les essais d'écrasement.
- elle possède une résistance en compression élevée à 2 h, qui est, selon la norme NF EN 196-1 de Mars 1990 concernant les essais en flexion et compression sur prisme 4 cm x 4 cm x 16 cm, supérieure à 40 MPa et peut atteindre 60 MPa ou plus.
- elle procure une bonne planéité des surfaçages réalisés sur les éprouvettes de compression.
- au cours des essais de résistance en compression d'éprouvettes de béton, elle permet le maintien d'un champ de contrainte au mieux uniforme ou au moins symétrique par rapport à l'axe des éprouvettes. Les résultats ont en effet permis d'établir que :
   - pour les compressions < 70 MPa, le surfaçage des éprouvettes au moyen de la composition selon l'invention donne des résultats équivalents à ceux obtenus par meulage des bases des éprouvettes, c'est-à-dire des résultats conformes à la réalité, avec une bonne régularité.
   - pour les compressions > 70 MPa, les résultats obtenus par surfaçage au moyen de la composition selon l'invention ne sont pas dispersés (les écarts entre les valeurs maximales et minimales mesurés sont faibles et n'excèdent pas 8 MPa). Ceci reste vrai pour des compressions élevées de l'ordre de 125 MPa.

La composition à base de soufre selon l'invention est plus particulièrement utile pour surfacer des éprouvettes de bétons dans les essais d'écrasement.

Néanmoins, elle peut également être employée pour réaliser des moulages rapides de pièces de dimension modérée du fait du retrait, c'est-à-dire d'épaisseur ou de diamètre de l'ordre de 20 cm ou inférieur, des calages, du colmatage ou pour réparer des fissures et des cavités apparues dans des ouvrages en béton.

Afin de faciliter sa compréhension, l'invention va maintenant être décrite plus précisément au travers d'exemples particuliers.

### Exemples 1 à 6

On a soumis à des essais d'écrasement des éprouvettes en béton dont certaines étaient surfacées avec des mortiers de soufre obtenus à partir des compositions à base de soufre reportées dans le tableau I suivant.

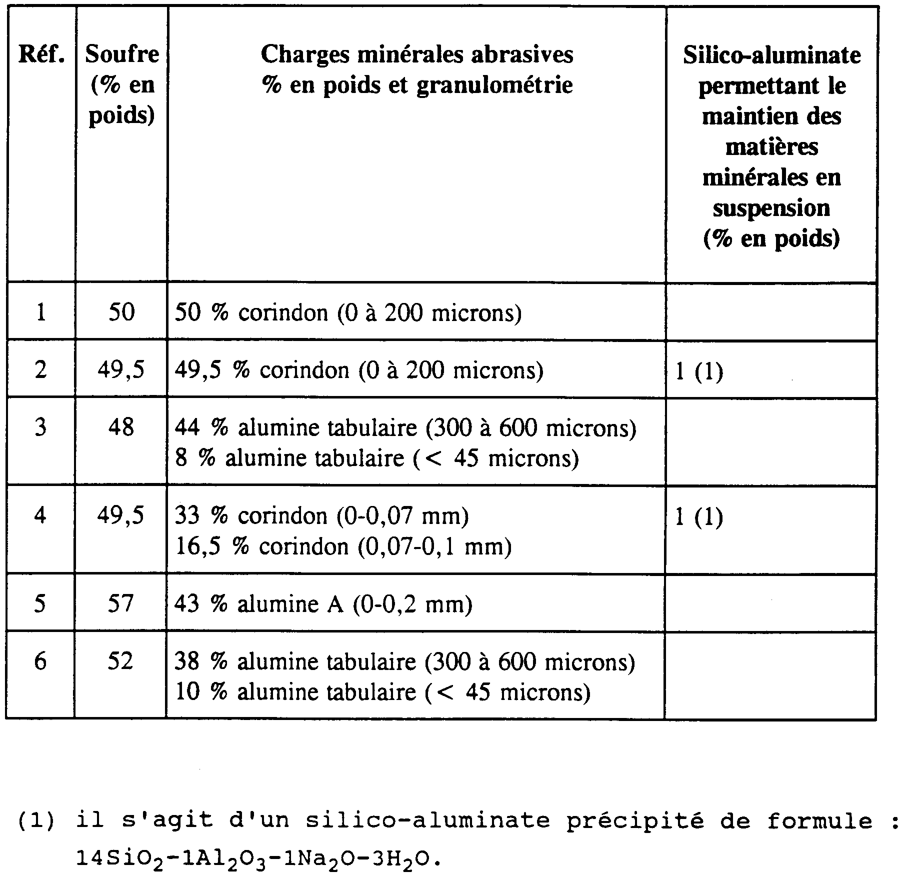

Les compositions précitées ont ensuite été évaluées conformément à la norme NF EN196-1 de Mars 1990 concernant les essais en flexion et compression sur prisme 4 cm x 4 cm x 16 cm.

Les résultats de cette évaluation sont reportés dans le tableau II suivant. Les compositions 1 à 7, conformes à l'invention, sont comparées à la composition normalisée (témoin) qui est composée de 60 % en poids de fleur de soufre et de 40 % en poids de sable de Fontainebleau de granulométrie inférieure à 0,5 mm.

| **Référence** | **Résistance 2 h Prisme 4 x 4 x 16 cm selon la norme NF-EN-196-1 de Mars 1990** | |
|---|---|---|
| | **Compression (MPa)** | **Flexion (MPa)** |
| Témoin | 28,1 | 4 |
| 1 | 54,2 | 9,6 |
| 2 | 56,5 | 7,7 |
| 3 | 51,3 | 6,3 |
| 4 | 61,1 | 8,4 |
| 5 | 48,3 | 6,2 |
| 6 | 51,9 | 6,6 |

Les compositions 1 à 6 offrent des résistances en compression élevées rapidement après leur mise en oeuvre. Elle sont par ailleurs faciles d'utilisation. Avec des bétons possédant une résistance en compression inférieure à 100 MPa, on constate qu'il n'y a pas de différence significative entre la rectification et le surfaçage réalisé avec les compositions 1 à 6.

Sur des bétons possédant une résistance en compression supérieure à 100 MPa, on obtient, avec ces compositions, des résultats très voisins de ceux obtenus par rectification. Les résultats offerts par ces compositions sont donc très satisfaisants.

### Exemple 7

### Surfaçage des éprouvettes pour essais en compression

Afin de connaître l'influence du mode de surfaçage sur les résultats en compression, des essais comparatifs ont été réalisés avec trois modes de surfaçage différents pour des compressions allant de 30 MPa à 130 MPa :
- rectification des surfaces par meulage,
- surfaçage normalisé au soufre (NF P18-416 de Décembre 1981), désigné ci-après sous "mélange normalisé" :
   - 60 % de fleur de soufre
   - 40 % de sable composé de sable de Fontainebleau, de granulométrie < 0,5 mm,
- surfaçage avec une composition conforme à l'invention réalisé dans les conditions de la norme NF P18-416 de Décembre 1981, excepté que la composition est chauffée entre 130 et 135°C pour leur conférer une meilleure maniabilité. Cette composition précisée ci-après est désignée sous "mélange A":
   - 48 % de soufre
   - 44 % d'alumine 28/48 mesh (0,600/0,300 mm)
   - 8 % d'alumine < 325 mesh (<0,045 mm).

### Performances mécaniques de ces deux produits

Ces deux produits ont été coulés rapidement en 4 x 4 x 16 cm, sans cristallisation interne et, dans chaque cas, nous avons déterminé, une heure après durcissement, la flexion et la compression sur trois prismes conformément à la norme NF-EN-196-1 de Mars 1990 concernant les essais en flexion et compression sur prisme 4 cm x 4 cm x 16 cm.

| **En MPa** | **Flexion** | **Compression** |
|---|---|---|
| mélange normalisé | 4,0 | 28,1 |
| composition selon l'invention | 6,3 | 51,3 |

On peut remarquer que :
- le mélange normalisé est moins performant,
- la compression obtenue grâce à la composition selon l'invention est élevée.

### Comparaison en surfaçage d'éprouvettes béton

Pour avoir un large éventail de compression, les six bétons suivants on été réalisés :

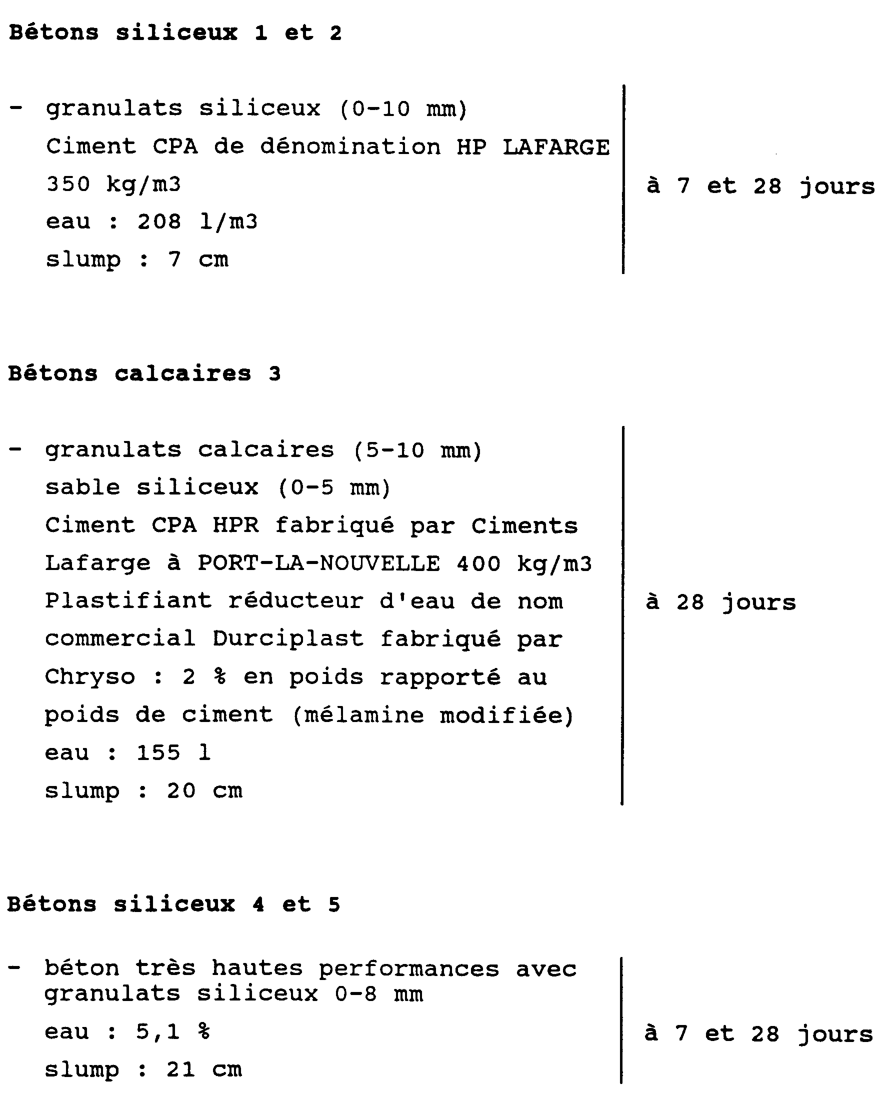

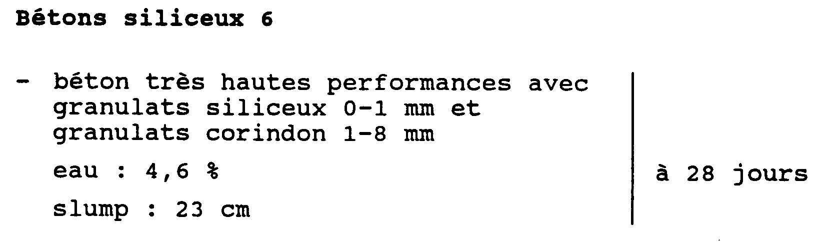

Remarque : slump correspond au test d'affaissement au cône d'Abraham, mesuré selon la norme NF P18-451 de Décembre 1981.

A chaque gâchée, il a été confectionné 15 cylindres de 11 cm de diamètre par 22 cm de hauteur dont :
- 3 ont été rectifiés par meulage,
- 3 ont été surfacés avec le mélange normalisé,
- 3 ont été surfacés avec le "mélange A" décrit dans cet exemple.

Chaque béton a été gâché deux fois, ce qui fait douze gâchées.

Après démoulage à 24 h, les cylindres ont été conservés jusqu'à rupture à 20°C et à 100 % d'humidité relative.

Les résultats obtenus sont regroupés dans le tableau ci-après.

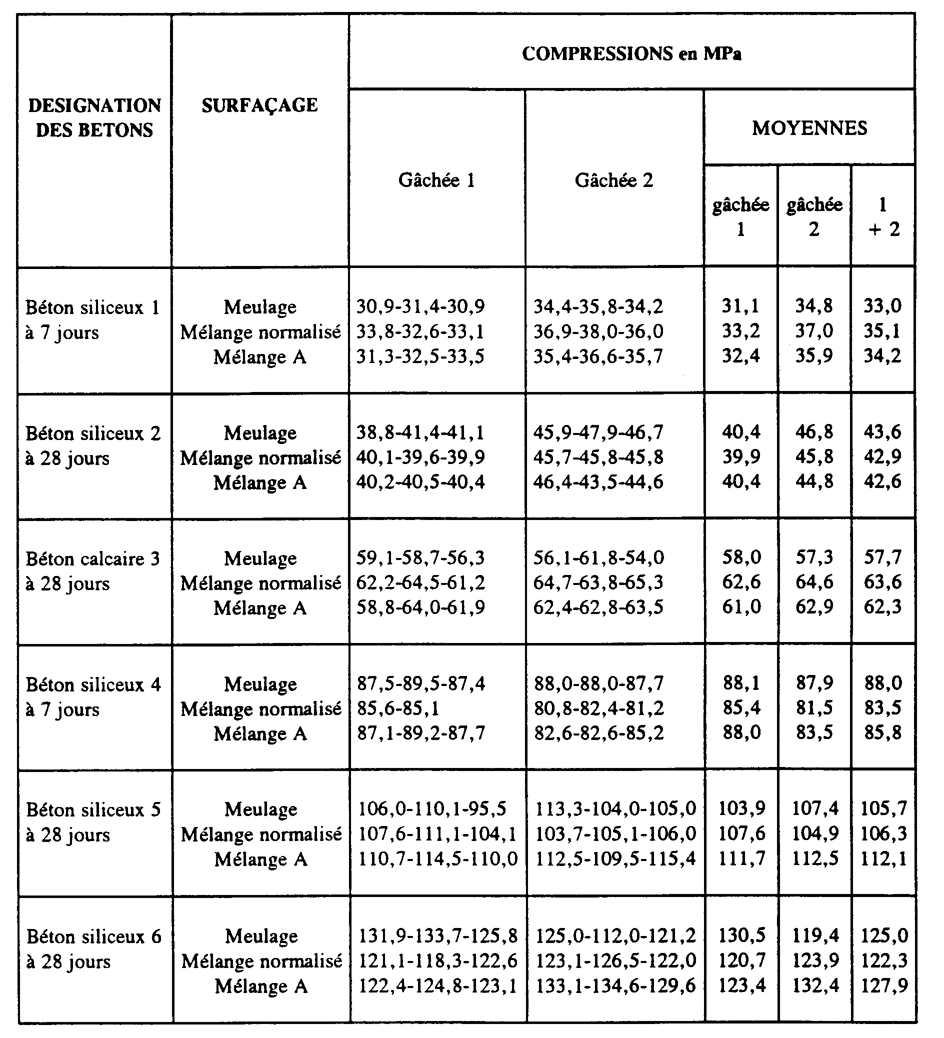

On constate que :
- pour les compressions < 70 MPa, les trois modes de surfaçage sont assez groupés.
- pour les compressions > 70 MPa :
- les écarts entre maximum et minimum sont plus faibles qu'on aurait pu le penser :
   - à 85 MPa écart 4,5 MPa
   - à 110 MPa écart 6,4 MPa
   - à 125 MPa écart 5,6 MPa
- le mélange normalisé donne pour les trois bétons de moins bonnes performances,
- le mélange A conduit à des performances élevées même à 100 et 125 MPa.

## Revendications

1. Composition à base de soufre, moulable à chaud, caractérisée en ce qu'elle comprend :
- du soufre, à raison d'au moins 40 % du poids de la composition globale ;
- et des charges minérales, parmi lesquelles on trouve au moins une charge minérale possédant une dureté de l'ordre de grandeur de celle du corindon ou supérieure (charge minérale (A)).

2. Composition selon la revendication 1, caractérisée en ce qu'elle comprend également un agent pour limiter la décantation des charges minérales quand la composition est à l'état fondu.

3. Composition selon la revendication 1 ou 2, caractérisée par le fait que la quantité de soufre est au moins égale à 45 % en poids.

4. Composition selon la revendication 3, caractérisée par le fait que la quantité de soufre est de 50 ± 2 % en poids.

5. Composition selon l'une des revendications 1 à 4, caractérisée par le fait que la charge minérale (A) est choisie, par ordre de préférence en partant de la charge procurant les meilleures performances :
• le corindon, dont le corindon brun qui est une alumine alpha impure,
• l'alumine tabulaire qui est une alumine alpha obtenue par frittage, la cuisson suivant une opération d'agglomération,
• l'alumine A ou alumine alpha obtenue par frittage,
• le cristalba qui est un corindon pur électro fondu,
• le carbure de silicium,
• ou des mélanges des charges minérales abrasives précitées.

6. Composition selon l'une des revendications 1 à 5, caractérisée par le fait que la proportion de l'agent permettant de limiter la décantation des charges minérales est inférieure à 5 % en poids rapporté à la quantité totale de la composition.

7. Utilisation d'une composition à base de soufre, moulable à chaud, réalisée selon l'une des revendications 1 à 6, pour le surfaçage des éprouvettes de béton dans des résistances en compression inférieures à 70 MPa dans les essais d'écrasement, cette composition à base de soufre répondant alors aux exigences suivantes :
• la proportion des charges minérales de dureté inférieure à celle du corindon (charges minérales (B)) est limitée ;
• la taille maximale des grains formant les charges minérales (A) et (B) est limitée à une valeur d'au plus 0,8 mm (inclus).

8. Utilisation d'une composition à base de soufre, moulable à chaud, réalisée selon l'une des revendications 1 à 6, pour le surfaçage des éprouvettes de béton de résistances en compression supérieures ou égales à 70 MPa dans les essais d'écrasement, cette composition à base de soufre répondant alors aux exigences suivantes :
• les charges minérales de dureté inférieure à celle du corindon sont exclues ;
• la taille maximale des grains formant la charge minérale (A) est limitée à une valeur d'au plus 0,8 mm (inclus).

9. Utilisation selon l'une des revendications 7 ou 8, caractérisée par le fait que la taille maximale des grains des charges minérales (A) et (B) est au plus égale à 0,5 mm (inclus).

10. Utilisation selon la revendication 9, caractérisée par le fait que la taille maximale des grains des charges minérales (A) et (B) est au plus égale à 0,3 mm (inclus).

11. Utilisation selon la revendication 10, caractérisée par le fait que la taille maximale des grains des charges minérales (A) et (B) est au plus égale à 0,2 mm (inclus).

12. Utilisation selon la revendication 11, caractérisée par le fait que la taille maximale des grains des charges minérales (A) et (B) est de préférence de l'ordre de 0,1 mm.

13. Utilisation d'une composition à base de soufre, moulable à chaud, réalisée selon l'une des revendications 1 à 6, pour le moulage rapide d'articles, le calage, le colmatage ou la réparation de fissures et de cavités.

## Claims

1. Sulphur-based composition, which can be moulded hot, characterised in that it comprises:
- sulphur, at the rate of at least 40% of the weight of the total composition;
- and mineral fillers, amongst which is included at least one mineral filler having a hardness of the order of magnitude of that of corundum or higher (mineral filler (A)).

2. Composition according to Claim 1, characterised in that it also comprises an agent for limiting the decantation of the mineral fillers when the composition is in the molten state.

3. Composition according to Claim 1 or 2, characterised by the fact that the quantity of sulphur is at least equal to 45% by weight.

4. Composition according to Claim 3, characterised by the fact that the quantity of sulphur is 50 ± 2 % by weight.

5. Composition according to one of Claims 1 to 4, characterised by the fact that the mineral filler (A) is chosen, by order of preference starting with the filler giving the best performances:
. corundum, namely brown corundum which is an impure alpha alumina,
. tabular alumina which is an alpha alumina obtained by fritting, the baking following an agglomeration operation,
. A alumina or alpha alumina obtained by fritting,
. cristalba which is a pure electro molten corundum,
. silicon carbide,
. or mixtures of the aforesaid abrasive mineral fillers.

6. Composition according to one of Claims 1 to 5, characterised by the fact that the proportion of the agent making it possible to limit the decantation of the mineral fillers is less than 5% by weight with respect to the total quantity of the composition.

7. Use of a sulphur-based composition, which can be moulded hot, made according to one of Claims 1 to 6, for the surfacing of concrete test-pieces with resistances to compression less than 70 MPa in crushing tests, this sulphur-based composition thus fulfilling the following requirements:
. the proportion of the mineral fillers of hardness less than that of corundum (mineral fillers (B)) is limited;
. the maximum size of the grains forming the mineral fillers (A) and (B) is limited to a value of at least 0.8 mm (inclusive).

8. Use of a sulphur-based composition, which can be moulded hot, made according to one of Claims 1 to 6, for the surfacing of concrete test-pieces with resistances to compression greater or equal to 70 MPa in crushing tests, this sulphur-based composition thus fulfilling the following requirements:
. mineral fillers of hardness less than that of corundum are excluded;
. the maximum size of the grains forming the mineral filler (A) is limited to a value of at the most 0.8 mm (inclusive).

9. Use according to one of Claims 7 or 8, characterised by the fact that the maximum size of the grains of the mineral fillers (A) and (B) is at the most equal to 0.5 mm (inclusive).

10. Use according to Claim 9, characterised by the fact that the maximum size of the grains of the mineral fillers (A) and (B) is at the most equal to 0.3 mm (inclusive).

11. Use according to Claim 10, characterised by the fact that the maximum size of the grains of the mineral fillers (A) and (B) is at the most equal to 0.2 mm (inclusive).

12. Use according to Claim 11, characterised by the fact that the maximum size of the grains of the mineral fillers (A) and (B) is preferably of the order of 0.1 mm.

13. Use of a sulphur-based composition, which can be moulded hot, made according to one of Claims 1 to 6, for the rapid moulding of articles, levelling, sealing, or the repair of cracks and cavities.

## Patentansprüche

1. Thermoformbare Zusammensetzung auf Schwefelbasis, dadurch gekennzeichnet, daß sie enthält:
- Schwefel im Anteil von mindestens 40 Gew.-% der Gesamtzusammensetzung
- und mineralische Füllstoffe, unter denen sich mindestens ein mineralischer Füllstoff befindet, der eine Härte in der Größenordnung der Härte des Korunds oder größer besitzt (mineralischer Füllstoff (A)).

2. Zusammensetzung nach Anspruch 1, dadurch gekennzeichnet, daß sie ebenfalls einen Wirkstoff enthält, um das Absetzen der mineralischen Füllstoffe zu begrenzen, wenn die Zusammensetzung im geschmolzenen Zustand ist.

3. Zusammensetzung nach Anspruch 1 oder 2, durch die Tatsache gekennzeichnet, daß die Schwefelmenge mindestens gleich 45 Gew.-% ist.

4. Zusammensetzung nach Anspruch 3, durch die Tatsache gekennzeichnet, daß die Schwefelmenge gleich 50 ± 2 Gew.-% ist.

5. Zusammensetzung nach einem der Ansprüche 1 bis 4, durch die Tatsache gekennzeichnet, daß der mineralische Füllstoff (A) unter den folgenden, nach der Bevorzugung angeordneten Füllstoffen gewählt ist, wobei mit dem Füllstoff begonnen wird, der die besten Leistungen bringt:
• Korund, darunter brauner Korund, der ein unreines Alpha-Aluminiumoxid ist,
• tafelförmiges Aluminiumoxid, das ein Alpha-Aluminiumoxid ist, das durch Sintern erhalten wird, wobei das Brennen auf eine Operation des Zusammenballens folgt,
• Aluminiumoxid A oder Alpha-Aluminiumoxid, erhalten durch Sintern,
• Cristalba, das ein reiner, elektrisch geschmolzener Korund ist,
• Siliciumcarbid
• oder Mischungen der vorher angeführten mineralischen abrasiven Füllstoffe.

6. Zusammensetzung nach einem der Ansprüche 1 bis 5, durch die Tatsache gekennzeichnet, daß der Anteil des Wirkstoffes, der es erlaubt, das Absetzen der mineralischen Füllstoffe zu begrenzen, kleiner als 5 Gew.-% bezogen auf die Gesamtmenge der Zusammensetzung ist.

7. Verwendung einer thermoformbaren Zusammensetzung auf Schwefelbasis, hergestellt nach einem der Ansprüche 1 bis 6, zur Oberflächenbearbeitung von Beton-Prüfkörpern mit Kompressionsbeständigkeiten unter 70 MPa in Kompressionsbeständigkeitsversuchen, wobei diese Zusammensetzung auf Schwefelbasis dann den folgenden Anforderungen entspricht:
• der Anteil von mineralischen Füllstoffen mit einer Härte unter der des Korunds (mineralische Füllstoffe (B)) ist begrenzt;
• die maximale Größe der Körner, welche die mineralischen Füllstoffe (A) und (B) bilden, ist auf einen Wert von höchstens 0,8 mm (einschließlich dieses Werts) begrenzt.

8. Verwendung einer thermoformbaren Zusammensetzung auf Schwefelbasis, hergestellt nach einem der Ansprüche 1 bis 6, zur Oberflächenbearbeitung von Beton-Prüfkörpern mit Kompressionsbeständigkeiten größer als oder gleich 70 MPa in Kompressionsbeständigkeitsversuchen, wobei diese Zusammensetzung auf Schwefelbasis dann den folgenden Anforderungen entspricht:
• mineralische Füllstoffe mit einer Härte unter der des Korunds sind ausgeschlossen;
• die maximale Größe der Körner, welche den mineralischen Füllstoff (A) bilden, ist auf einen Wert von höchstens 0,8 mm (einschließlich dieses Werts) begrenzt.

9. Verwendung nach einem der Ansprüche 7 oder 8, durch die Tatsache gekennzeichnet, daß die maximale Größe der Körner der mineralischen Füllstoffe (A) und (B) höchstens gleich 0,5 mm (einschließlich dieses Werts) ist.

10. Verwendung nach Anspruch 9, durch die Tatsache gekennzeichnet, daß die maximale Größe der Körner der mineralischen Füllstoffe (A) und (B) höchstens gleich 0,3 mm (einschließlich dieses Werts) ist.

11. Verwendung nach Anspruch 10, durch die Tatsache gekennzeichnet, daß die maximale Größe der Körner der mineralischen Füllstoffe (A) und (B) höchstens gleich 0,2 mm (einschließlich dieses Werts) ist.

12. Verwendung nach Anspruch 11, durch die Tatsache gekennzeichnet, daß die maximale Größe der Körner der mineralischen Füllstoffe (A) und (B) vorzugsweise von der Größenordnung 0,1 mm ist.

13. Verwendung einer thermoformbaren Zusammensetzung auf Schwefelbasis, hergestellt nach einem der Ansprüche 1 bis 6, für das schnellen Gießen von Gegenständen, das Einpassen, das Abdichten oder das Ausbessern von Rissen oder Hohlräumen.
